(19) Europäisches Patentamt
European Patent Office
Office européen des brevets

(11) EP 3 250 669 B1

(12) # EUROPÄISCHE PATENTSCHRIFT

(45) Veröffentlichungstag und Bekanntmachung des Hinweises auf die Patenterteilung:
**07.07.2021 Patentblatt 2021/27**

(21) Anmeldenummer: **15790156.2**

(22) Anmeldetag: **05.11.2015**

(51) Int Cl.:
*C11D 3/386* (2006.01) *C11D 3/20* (2006.01)
*C11D 1/72* (2006.01) *C11D 11/00* (2006.01)
*C11D 17/04* (2006.01)

(86) Internationale Anmeldenummer:
**PCT/EP2015/075799**

(87) Internationale Veröffentlichungsnummer:
**WO 2016/119933 (04.08.2016 Gazette 2016/31)**

(54) **SAURES FLÜSSIGKOMPAKTWASCHMITTEL ENTHALTEND HYDROXYCARBONSÄURE, NIOTENSID UND ENZYM**

ACID LIQUID COMPACT WASHING AGENT CONTAINING HYDROXYCARBOXYLIC ACID, NON-IONIC SURFACTANT AND AN ENZYME

LESSIVE LIQUIDE ACIDE À FAIBLE TENEUR EN EAU, CONTENANT DE L’ACIDE HYDROXYCARBOXYLIQUE, UN TENSIOACTIF NEUTRE ET UNE ENZYME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2015 DE 102015201702**

(43) Veröffentlichungstag der Anmeldung:
**06.12.2017 Patentblatt 2017/49**

(73) Patentinhaber: **Henkel AG & Co. KGaA**
**40589 Düsseldorf (DE)**

(72) Erfinder:
- **MEINE, Georg**
  **40822 Mettmann (DE)**
- **O’CONNELL, Timothy**
  **40547 Düsseldorf (DE)**

(56) Entgegenhaltungen:
**WO-A1-92/19711** **WO-A1-95/02044**
**WO-A1-99/50380** **WO-A1-2012/149325**
**WO-A1-2014/018309** **WO-A1-2014/162001**
**DE-A1- 19 606 619** **GB-A- 2 373 254**
**US-A1- 2013 184 195**

- **anonymous: "Acid Protease", Encyclopedia.com , Januar 2004 (2004-01), XP002753468, Gefunden im Internet: URL:http://www.encyclopedia.com/doc/1O6-acidprotease.html [gefunden am 2016-01-26]**
- **"Papain (Sigma-Aldricht)", , Retrieved from the Internet: URL:https://www.sigmaaldrich.com/life-science/metabolomics/enzyme-explorer/analytical-enzymes/papain.html [retrieved on 2018-10-17]**
- **Ruchita Dubey, Soumya Reddy and N.Y.S. Murthy: "Optimization of Activity of bromelain", Asian J. Chem., vol. 24, no. 4 1 October 2014 (2014-10-01), 2012, pages 1429-1431, Retrieved from the Internet: URL:http://www.asianjournalofchemistry.co.in/user/journal/viewarticle.aspx?ArticleID=24_4_4 [retrieved on 2018-10-17]**
- **Eiji Ichishima & Fumihiko Yoshida: "Molecular Weight of Acid Proteinase of Aspergillus saitoi", Nature, vol. 207 31 July 1965 (1965-07-31), pages 525-526, Retrieved from the Internet: URL:https://www.nature.com/articles/207525 a0 [retrieved on 2018-10-17]**
- **, Retrieved from the Internet: URL:http://commonchemistry.org/ChemicalDetail.aspx?ref=9001-92-7 [retrieved on 2018-10-17]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 250 669 B1

- , Retrieved from the Internet: URL:https://calzyme.com/commerce/catalog/spcategory.jsp?category_id=1094 [retrieved on 2018-10-17]

## Beschreibung

Die vorliegende Anmeldung richtet sich auf wasserfreie bis wasserarme flüssige Waschmittelzusammensetzungen mit saurem pH-Wert, die mindestens eine niedermolekulare Hydroxycarbonsäure, mindestens ein nichtionisches Tensid und mindestens ein Enzym mit einem pH-Optimum von pH < 7 enthalten, die Verwendung solcher Flüssigwaschmittel zur Entfernung von Anschmutzungen, insbesondere solchen, die auf Bestandteilen und Rückständen von Deodorantien, aber auch Rost, Beeren, Tee und Rotwein, beruhen, sowie auf ein Waschverfahren, in dem ein solches Mittel verwendet wird.

Vom Verbraucher verwendete Deodorantien bilden mit Absonderungen der menschlichen Haut schwerlösliche, gelbliche Rückstände auf getragenen Textilien, die im Wesentlichen Aluminiumhydroxychlorid, Parfümölbestandteile, Wachse wie z.B. Myristylmyristat und menschliche Bestandteile der Haut und niedermolekulare Fettsäuren des Schweißes enthalten. Weitere schwerlösliche Anschmutzungen auf Textilien können von Rost und Lebensmittelrückständen, insbesondere von Beeren, Tee und Rotwein stammen.

Herkömmliche Flüssigwaschmittel basieren auf Tensidmischungen, die beispielsweise Seifen, Fettalkoholethersulfate und -ethoxylate umfassen, wobei die Gesamttensidmengen in Flüssigwaschmitteln etwa 20 Gew.% und in konzentrierten Mitteln etwa 50 Gew.% betragen. Allerdings entfernen solche bekannten Flüssigwaschmittel derartige schwerlösliche Rückstände nicht oder nur ungenügend. Es ist daher ein generelles Bestreben, Flüssigwaschmittel mit verbesserter Reinigungsleistung insbesondere auf Anschmutzungen, die auf Bestandteilen und Rückständen von Deodorantien, aber auch auf Rost, Beeren, Tee und Rotwein basieren, bereitzustellen. Dabei soll die Reinigungsleistung der Waschmittel auf enzymsensitiven Anschmutzungen ebenso vorhanden sein.

Chang et al. (Biochem. Mol. Biol. Int. Mai 1995 (1995-05): 185-193) beschreiben die Reinigung und Charakterisierung einer alpha-Amylase aus *Aspergillus oryzae* ATCC 76080. Die Amylase hat ein pH Optimum zwischen 4 und 5, ein Temperaturoptimum bei 50°C und einen Km Wert von 0.22% für die Hydrolyse von Stärke, wobei aber 80% der Enzymaktivität nach Inkubation bei 50°C für 30 Minuten verloren gehen.

WO2012149325 betrifft ein Mannanaseenzym mit einem pH-Optimum von pH 5 für Wasch- oder Reinigungsmittel.

Es wurde nun überraschend gefunden, dass sich in Flüssigwaschmittel, die wasserarm bis wasserfrei sind, 10 bis 30 Gew.%, insbesondere 15 bis 25 Gew.% niedermolekularer Hydroxycarbonsäure und 20 bis 55 Gew.%, insbesondere 35 bis 50 Gew.% mindestens eines nichtionischen Tensides enthalten, und einen pH-Wert im Bereich 2 bis 5, besonders bevorzugt einen pH von 4,6 aufweisen, Enzyme mit einem pH-Optimum von pH < 7, stabiler einarbeiten lassen und dieses Waschmittel eine erhöhte Reinigungsleistung, insbesondere an Anschmutzungen, die auf Bestandteilen und Rückständen von Deodorantien basieren aber auch auf Rost, Beeren, Tee und Rotweinrückständen, aufweisen.

In einem ersten Aspekt betrifft die vorliegende Erfindung daher ein Flüssigwaschmittel, das bezogen auf das Gesamtgewicht des Flüssigwaschmittels

(i) 10 bis 30 Gew.%, insbesondere 15 bis 25 Gew.% mindestens einer Hydroxycarbonsäure mit 2 bis 8 Kohlenstoffatomen und
(ii) 20 bis 55 Gew.%, insbesondere 35 bis 50 Gew.% mindestens eines nichtionischen Tensides enthält,
(iii) 0 bis 20 Gew.-% Wasser,
(iv) mindestens ein Enzym mit einem pH-Optimum von pH < 7

wobei das Flüssigwaschmittel einen pH-Wert im Bereich 2 bis 5 aufweist.

In einem weiteren Aspekt richtet sich die vorliegende Erfindung auf die Verwendung des hierin beschriebenen Flüssigwaschmittels zum Waschen von Textilien, insbesondere zur Entfernung von Anschmutzungen, die auf Bestandteilen und Rückständen von Deodorantien, Rost, Beeren, Tee und Rotwein basieren.

Schließlich betrifft die vorliegende Erfindung in einem weiteren Aspekt ein Waschverfahren zum Waschen von Textilien, wobei das wasserarme bis wasserfreie Flüssigwaschmittel wie hierin beschrieben verwendet wird.

Unter Reinigungsleistung (Waschkraft) wird im Rahmen der Erfindung die Entfernung von einer oder mehreren Anschmutzungen, insbesondere Wäscheanschmutzungen, verstanden, die bleichesensitiv, enzymsensitiv oder tensidsensitiv, insbesondere tensidsensitiv sind. Die Entfernung kann über eine Aufhellung der Anschmutzung sowohl messtechnisch erfasst als auch visuell beurteilt werden.

Die hierin beschriebenen Waschmittel können Waschmittel für Textilien oder Naturfasern sein. Zu den Waschmitteln im Rahmen der Erfindung zählen ferner Waschhilfsmittel, die bei der manuellen oder maschinellen Textilwäsche zum eigentlichen Waschmittel zudosiert werden, um eine weitere Wirkung zu erzielen oder um eine Wirkung zu verstärken. Fernerzählen zu Waschmitteln im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten

Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Durch den Einsatz des hierin beschriebenen Flüssigwaschmittels lassen sich Anschmutzungen, die auf Bestandteilen und Rückständen von Deodorantien basieren, aber auch Anschmutzungen mit Rost, Beeren, Tee und Rotwein entfernen. Durch eine Vorbehandlung des betreffenden Textils an der verschmutzen Stelle ist die Fleckenentfernung deutlich verbessert. Eine gewisse Feuchtigkeit des Textils verstärkt die Wirkung der Zitronensäure. Trotz des niedrigen pH-Werts lässt sich das erfindungsgemäße Enzym stabil in das besagte Waschmittel einarbeiten.

Insbesondere enthalten erfindungsgemäße wasserarme Zusammensetzungen bevorzugt 5 bis 20 Gew.% Wasser, bevorzugter 5 bis 15 Gew.% Wasser, besonders bevorzugt 10 bis weniger als 15 Gew.% Wasser. Solche bevorzugten Zusammensetzungen werden als "wasserarm" bezeichnet.

Wie hierin verwendet bedeutet "wasserfrei", dass eine Zusammensetzung weniger als 5 Gew.%, insbesondere weniger als 4 Gew.%, vorzugsweise weniger als 3 Gew.% Wasser enthält. Solche wasserfreien Zusammensetzungen sind erfindungsgemäß besonders bevorzugt.

Der Wassergehalt wie hierin definiert bezieht sich auf den mittels der Karl Fischer Titration ermittelten Wassergehalt (Angewandte Chemie 1935, 48, 394-396; ISBN 3-540-12846-8 Eugen Scholz).

"Flüssig", wie hierin in Bezug auf das erfindungsgemäße Waschmittel verwendet, schließt alle bei Standardbedingungen (20 °C, 1013 mbar) fließfähigen Zusammensetzung im flüssigen Aggregatzustand ein und erfasst insbesondere auch Gele und pastöse Zusammensetzungen. Insbesondere schließt der Begriff auch Nicht-Newtonsche Flüssigkeiten, die eine Fließgrenze besitzen, ein.

Alle im Zusammenhang mit den hierin beschriebenen Bestandteilen des Waschmittels angegeben Mengenangaben beziehen sich, sofern nichts anderes angegeben ist, auf Gew.% jeweils bezogen auf das Gesamtgewicht des Waschmittels. Des Weiteren beziehen sich derartige Mengenangaben, die sich auf mindestens einen Bestandteil beziehen, immer auf die Gesamtmenge dieser Art von Bestandteil, die im Waschmittel enthalten ist, sofern nicht explizit etwas anderes angegeben ist. Das heißt, dass sich derartige Mengenangaben, beispielsweise im Zusammenhang mit "mindestens einem nichtionischen Tensid", auf die Gesamtmenge von nichtionischen Tensiden die im Waschmittel enthalten ist, beziehen.

"Mindestens ein", wie hierin verwendet, bezieht sich auf 1 oder mehr, beispielsweise 1, 2, 3, 4, 5, 6, 7, 8, 9 oder mehr. Im Zusammenhang mit Bestandteilen der hierin beschriebenen Zusammensetzungen bezieht sich diese Angabe nicht auf die absolute Menge an Molekülen sondern auf die Art des Bestandteils. "Mindestens ein nichtionisches Tensid" bedeutet daher beispielsweise ein oder mehrere verschiedene nichtionische Tenside, d.h. eine oder mehrere verschiedene Arten von nichtionischen Tensiden. Zusammen mit Mengenangaben beziehen sich die Mengenangaben auf die Gesamtmenge der entsprechend bezeichneten Art von Bestandteil, wie bereits oben definiert.

Wenn hierin auf den pH-Wert der Zusammensetzung/des Waschmittels Bezug genommen wird, beziehen sich die angegebenen Werte immer auf den pH-Wert einer 1%igen (Gew.-%) Lösung des Waschmittels in destilliertem Wasser bei 25 °C. Dies gilt explizit nicht für den pH-Wert des pH-Optimums (*vide infra*).

Das wasserarme bis wasserfreie Flüssigwaschmittel umfasst 10 bis 30 Gew.%, insbesondere 15 bis 25 Gew.% mindestens einer Hydroxycarbonsäure mit 2 bis 8 Kohlenstoffatomen, wobei aliphatische Hydroxycarbonsäuren mit 2 bis 8 Kohlenstoffatomen bevorzugt sind.

Bei Einsatz chiraler Hydroxycarbonsäuren wie Milchsäure oder Hydroxybernsteinsäure, können sowohl das Racemat als auch die reinen D- oder L-Hydroxycarbonsäuren oder deren Mischungen in dem erfindungsgemäßen Flüssigwaschmittel verwendet werden.

Besonders bevorzugt enthält das erfindungsgemäße Flüssigwaschmittel mindestens eine Hydroxycarbonsäure ausgewählt aus mindestens einem Vertreter der Gruppe, die gebildet wird aus Zitronensäure, Milchsäure, Weinsäure, Hydroxybernsteinsäure, Salicylsäure.

Ganz besonders bevorzugt enthält das erfindungsgemäße Flüssigwaschmittel 10 bis 30 Gew.%, insbesondere 15 bis 25 Gew.% mindestens einer, aliphatischen Hydroxycarbonsäure mit 3 bis 6 Kohlenstoffatomen, wobei besagte Hydroxycarbonsäure linear oder verzweigt ist.

Ganz besonders bevorzugt enthält das erfindungsgemäße Flüssigwaschmittel 10 bis 30 Gew.%, insbesondere 15 bis 25 Gew.% mindestens einer aliphatischen Hydroxycarbonsäure mit 3 bis 6 Kohlenstoffatomen ausgewählt aus Zitronensäure, Milchsäure oder deren Gemisch.

Unter Milchsäure ist sowohl das Racemat als auch die optisch aktiven D/L-Varianten zu verstehen.

Als Zitronensäure kann sowohl wasserfreie Zitronensäure als auch Zitronensäuremonohydrat oder Gemische davon eingesetzt werden. Vorzugsweise wird Zitronensäuremonohydrat verwendet.

Darüber hinaus sind Mischungen von Zitronensäure und Milchsäure gut geeignet wobei unter Einhaltung der besagten Gesamtmenge besagter Hydroxysäuren die Menge von Milchsäure von 1 bis 10 Gew.-% und die Menge der Zitronensäure von 4 bis 29 Gew.-% beträgt.

Falls eine Kombination aus Milchsäure und Zitronensäure eingesetzt wird, ist es erfindungsgemäß bevorzugt das Gewichtsverhältnis von Milchsäure zu Zitronensäure in einem Gewichtsverhältnisbereich von 1 zu 2 bis 1 zu 10,

besonders bevorzugt von 1 zu 3 bis 1 zu 7, ganz besonders bevorzugt von 1 zu 4 bis 1 zu 6, einzustellen.

Dabei ist es wiederum am bevorzugtesten, die unter Einhaltung der besagten Gesamtmenge besagter Hydroxysäuren die Menge von Milchsäure von 1 bis 10 Gew.-% und die Menge der Zitronensäure von 4 bis 29 Gew.-% zu wählen und das Gewichtsverhältnis von Milchsäure zu Zitronensäure in einem Gewichtsverhältnisbereich von 1 zu 2 bis 1 zu 10, besonders bevorzugt von 1 zu 3 bis 1 zu 7, ganz besonders bevorzugt von 1 zu 4 bis 1 zu 6, einzustellen.

Ferner enthält das Flüssigwaschmittel nach der vorliegenden Erfindung 20 bis 55 Gew.%, insbesondere 35 bis 50 Gew.% mindestens eines nichtionischen Tensides bezogen auf die Gesamtmenge des Waschmittels. Geeignete nichtionische Tenside umfassen alle bekannten, in Waschmitteln üblicherweise eingesetzten Tenside, insbesondere solche, die ausgewählt sind aus der Gruppe bestehend aus Alkylglykolethern, alkoxylierten Fettalkoholen, alkoxylierten OxoAlkoholen, alkoxylierten Fettsäurealkylestern, Fettsäureamiden, alkoxylierten Fettsäureamiden, Polyhydroxyfettsäureamiden, Alkylphenolpolyglycolethern, Aminoxiden, Alkyl(poly)glucosiden und Mischungen daraus.

In verschiedenen Ausführungsformen der Erfindung enthalten die hierin beschriebenen Waschmittel mindestens ein Fettalkoholalkoxylat mit der nachstehenden Formel (I) umfassen

$$R^1\text{-O-(AO)}_m\text{-H} \qquad \text{(I)},$$

wobei

$R^1$ ein linearer oder verzweigter Alkylrest ist,
AO eine Ethylenoxid- (EO) oder Propylenoxid- (PO) Gruppierung ist,
m eine ganze Zahl von 1 bis 50 ist.

In der vorstehenden Formel steht $R^1$ für einen linearen oder verzweigten, subtituierten oder unsubstituierten Alkylrest. In einer bevorzugten Ausführungsform der vorliegenden Erfindung ist $R^1$ ein linearer oder verzweigter Alkylrest mit 5 bis 30 C-Atomen, vorzugsweise mit 7 bis 25 C-Atomen und insbesondere mit 10 bis 19 C-Atomen. Bevorzugte Reste $R^1$ sind ausgewählt aus Decyl-, Undecyl-, Dodecyl-, Tridecyl-, Tetradecyl, Pentadecyl-, Hexadecyl-, Heptadecyl-, Octadecyl-, Nonadecylresten und deren Mischungen, wobei die Vertreter mit gerader Anzahl an C-Atomen bevorzugt sind. Besonders bevorzugte Reste $R^1$ sind abgeleitet von Fettalkoholen mit 12 bis 19 C-Atomen, beispielsweise von Kokosfettalkohol, Talgfettalkohol, Lauryl-, Myristyl-, Cetyl- oder Stearylalkohol oder von Oxoalkoholen mit 10 bis 19 C-Atomen.

AO ist eine Ethylenoxid- (EO) oder Propylenoxid- (PO) Gruppierung, vorzugsweise eine Ethylenoxidgruppierung. Der Index m ist eine ganze Zahl von 1 bis 50, vorzugsweise 2 bis 20 und bevorzugt 2 bis 10. Insbesondere ist m 3, 4, 5, 6 oder 7. Das erfindungsgemäße Mittel kann Mischungen von nichtionischen Tensiden enthalten, die verschiedene Ethoxylierungsgrade aufweisen. Bevorzugt sind Tenside mit Alkoxylierungs-/Ethoxylierungsgraden von mindestens 5.

Zusammenfassend sind besonders bevorzugte Fettalkoholalkoxylate solche der Formel

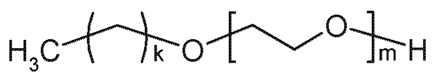

(C-1)

mit k = 9 bis 17, m = 3, 4, 5, 6 oder 7. Ganz besonders bevorzugte Vertreter sind Fettalkohole mit 10 bis 18 C-Atomen und mit 7 EO (k = 11-17, m = 7 in Formel C-1).

Solche Fettalkoholethoxylate sind unter den Verkaufsbezeichnungen Dehydol® LT7 (Cognis), Lutensol® AO7 (BASF), Lutensol® M7 (BASF) und Neodol® 45-7 (Shell Chemicals) erhältlich.

Die oben genannten Fettalkoholethoxylate haben vorzugsweise Ethoxylierungsgrade von mindestens 3, besonders bevorzugt mindestens 5, wiederum vorzugsweise von 7. Derartige Fettalkoholethoxylate können allein, als Mischungen solcher Fettalkoholethoxylate oder auch als Mischungen mit niedriger ethoxylierten Fettalkoholethoxylaten, wie beispielsweise Lutensol® A03 (BASF), eingesetzt werden. In solchen Mischungen ist es bevorzugt, dass die Fettalkoholethoxylate mit Ethoxylierungsgraden von mindestens 5, vorzugsweise 7, mindestens 50 Gew.%, vorzugsweise mindestens 75 Gew.% der Gesamtmenge an Fettalkoholethoxylaten ausmachen.

Weitere einsetzbare, nichtionische Tenside können beispielsweise sein

- Polyolfettsäureester, wie beispielsweise das Handelsprodukt Hydagen® HSP (Cognis) oder Sovermol - Typen (Cognis),
- alkoxylierte Triglyceride,
- alkoxylierte Fettsäurealkylester der Formel $R^3CO\text{-(OCH2CHR}^4)_wOR^5$, in der $R^3CO$ für einen linearen oder verzweigten, gesättigten und/oder ungesättigten Acylrest mit 6 bis 22 C-Atomen, $R^4$ für Wasserstoff oder Methyl und $R^5$ für lineare oder verzweigte Alkylreste mit 1 bis 4 C-Atomen steht und w 1 bis 20 ist,

- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide, und
- Alkyl(poly)glycoside der Formel $R^2O\text{-}[G]_p$,

in der $R^2$ ein lineares oder verzweigtes Alkyl mit 12 bis 16 C-Atomen, G ein Zuckerrest mit 5 oder 6 C-Atomen, insbesondere Glucose, und der Index p 1 bis 10 ist.

Das mindestens eine nichtionische Tensid, das erfindungsgemäß eingesetzt wird, weist vorzugsweise einen HLB-Wert von mindestens 5 aber höchstens 18, vorzugsweise von 8 bis 17 und besonders bevorzugt von 9 bis 16 auf.

Der Begriff "HLB" (hydrophilic-lipophilic balance) definiert den hydrophilen und lipophilen Anteil entsprechender Substanzklassen (hierTenside) in einem Wertebereich von 1 bis 20 nach folgender Formel (Griffin, Classification of surface active agents by HLB, J. Soc. Cosmet. Chem. 1, 1949):

$$HLB = 20 \times (1-(M_1/M))$$

mit M = Molmasse des gesamten Moleküls
und $M_1$ = Molmasse des lipophilen Anteils des Moleküls

Niedrige HLB-Werte ($\geq 1$) beschreiben lipophile Stoffe, hohe HLB Werte ($\leq 20$) beschreiben hydrophile Stoffe. So haben beispielsweise Entschäumer typischerweise HLB-Werte im Bereich von 1,5 bis 3 und sind unlöslich in Wasser. Emulgatoren für W/O-Emulsionen haben typischerweise HLB-Werte im Bereich von 3 bis 8, wohingegen Emulgatoren für O/W-Emulsionen typischerweise HLB-Werte im Bereich von 8 bis 18 aufweisen. Waschaktive Substanzen haben typischerweise HLB-Werte im Bereich von 13 bis 15 und Lösungsvermittler Werte im Bereich von 12 bis 18.

In verschiedenen Ausführungsformen der Erfindung weisen die nichtionischen Tenside, die erfindungsgemäß eingesetzt werden, kritische Mizellbildungskonzentrationen von $10^{-3}$ mol/L oder mehr, insbesondere $10^{-2}$ mol/L oder mehr auf. Im Einklang mit dem üblichen Verständnis auf diesem Gebiet, gibt die kritische Mizellbildungskonzentration (CMC) die Tensidkonzentration an, bei dersich Mizellen bilden und das gesamte zusätzliche Tensid, das zugegeben wird, in die Mizellen wandert.

Des Weiteren kann das Waschmittel mindestens ein anionisches Tensid, insbesondere ein Alkylbenzolsulfonat und/oder ein Alkylethersulfat und/oder eine Fettsäureseife, enthalten. Derartige anionische Tenside können in Form ihrer Salze aber auch als die korrespondierenden Säuren eingesetzt werden.

Als Tenside vom Sulfonat-Typ kommen dabei vorzugsweise Alkylbenzolsulfonate, Olefinsulfonate, d.h. Gemische aus Alken- und Hydroxyalkansulfonaten sowie Disulfonaten, wie man sie beispielsweise aus Monoolefinen mit 12 bis 18 C-Atomen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate mit 12 bis 18 C-Atomen und die Ester von $\alpha$-Sulfofettsäuren (Estersulfonate), zum Beispiel die $\alpha$-sulfonierten Methylester der hydrierten Kokos-, Palmkern- oder Talgfettsäuren.

Alkylbenzolsulfonate sind vorzugsweise ausgewählt aus linearen oder verzweigten Alkylbenzolsulfonaten der Formel

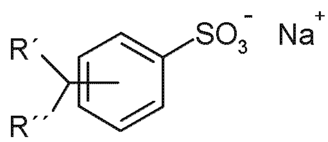
,

in der R' und R" unabhängig Wasserstoff oder Alkyl sind und zusammen 9 bis 19, vorzugsweise 9 bis 15 und insbesondere 9 bis 13 C-Atome enthalten. Ein ganz besonders bevorzugter Vertreter ist Natriumdodecylbenzylsulfonat. In verschiedenen bevorzugten Ausführungsformen kommen die korrespondierenden Säuren der oben genannten Alkylbenzolsulfonate zum Einsatz.

Als Alk(en)ylsulfate werden die Salze der Schwefelsäurehalbester der Fettalkohole mit 12 bis 18 C-Atomen, beispielsweise aus Kokosfettalkohol, Talgfettalkohol, Lauryl-, Myristyl-, Cetyl- oder Stearylalkohol oder der Oxo-Alkohole mit 10 bis 20 C-Atomen und diejenigen Halbester sekundärer Alkohole dieser Kettenlängen bevorzugt. Aus waschtechnischem Interesse sind die Alkylsulfate mit 12 bis 16 C-Atomen und Alkylsulfate mit 12 bis 15 C-Atomen sowie Alkylsulfate

mit 14 und 15 C-Atomen bevorzugt. Auch 2,3-Alkylsulfate sind geeignete anionische Tenside.

Auch Alkylethersulfate mit der Formel

$$R^1\text{-O-(AO)}_n\text{-SO}_3^-\ X^+$$

sind geeignet. In dieser Formel steht $R^1$ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste $R^1$ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste $R^1$ sind abgeleitet von Fettalkoholen mit 12 bis 18 C-Atomen, beispielsweise von Kokosfettalkohol, Talgfettalkohol, Lauryl-, Myristyl-, Cetyl- oder Stearylalkohol oder von Oxoalkoholen mit 10 bis 20 C-Atomen.

AO steht für eine Ethylenoxid- (EO) oder Propylenoxid- (PO) Gruppierung, vorzugsweise für eine Ethylenoxidgruppierung. Der Index n ist eine ganze Zahl von 1 bis 50, vorzugsweise von 1 bis 20 und insbesondere von 2 bis 10. Ganz besonders bevorzugt ist n 2, 3, 4, 5, 6, 7 oder 8. X ist ein einwertiges Kation oder den n-ten Teil eines n-wertigen Kations, bevorzugt sind dabei die Alkalimetallionen und darunter $Na^+$ oder $K^+$, wobei $Na^+$ äußerst bevorzugt ist. Weitere Kationen X+ können ausgewählt sein aus $NH4^+$, ½ $Zn^{2+}$,½ $Mg^{2+}$,½ $Ca^{2+}$½ $Mn^{2+}$, und deren Mischungen.

Besonders bevorzugte Waschmittel enthalten ein Alkylethersulfat ausgewählt aus Fettalkoholethersulfaten der Formel A-1

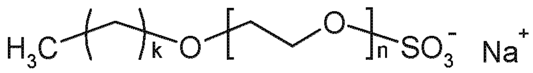

(A-1)

mit k = 11 bis 19, n = 2, 3, 4, 5, 6, 7 oder 8. Ganz besonders bevorzugte Vertreter sind Na Fettalkoholethersulfate mit 12 bis 18 C-Atomen und 2 EO (k = 11 bis 13, n = 2 in Formel A-1). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Weiterhin können Alkyl- und/oder Alkenyl-Oligoglykosidcarboxylate, -sulfate, -phosphate und/oder-isethionate, die sich von Alkyl- und/oder Alkenyloligoglykosiden der allgemeinen Formel (A-2)

R6-O-(G) (A-2)

ableiten, eingesetzt werden, wobei

- $R^6$ Alkyl mit 6 bis 22 C-Atomen oder Alkenyl mit 6 bis 22 C-Atomen,
- G Glykosideinheit, die sich von einem Zucker mit 5 oder 6 Kohlenstoffatomen ableitet,
- p Zahl von 1 bis 10.

Die Verwendung von Alkyl- und/oder Alkenyl-Oligoglykosidcarboxylaten, -sulfaten, -phosphaten und/oder -isethionaten in Textilbehandlungsmitteln wie beispielsweise Waschmitteln oder Weichspülern führt unter anderem zu einer verbesserten Farbstabilität gefärbter Textilien und zu einer verbesserten Reinigungsleistung des Waschmittels. Bei Einsatz in flüssigen Mitteln, insbesondere flüssigen Waschmitteln, wird verglichen mit herkömmlichen Farbübertragungsinhibitoren, die Lagerstabilität des Mittels erhöht. Es können Alkyloligoglykosidcarboxylate, -sulfate, -phosphate und/oder -isethionate, eingesetzt werden, bevorzugt Alkyloligoglykosidcarboxylate oder -phosphate, insbesondere Alkylglykosidcarboxylate.

In den Alkyl- und/oder Alkenyl-Oligoglykosiden ist vorzugsweise in mindestens einem der Reste G mindestens eine Hydroxylgruppe durch $-O-C_{1-12}$-Alkenyl-COOM, $-OSO_3M$, $-OP(O)(OM)_2$ oder $-O-CH_2-CH_2-SO_3M$, $O-CH_2-CH_2-O-SO_3M$, jeweils mit M = H, Alkalimetall oder $NH_4$ ersetzt. Dabei wird besonders bevorzugt ein Alkyloligoglykosid-Carboxylat eingesetzt, in dem $-O-C_{1-12}$-Alkylen-COOM oder $-O(CH_2-)_nCOOM$ mit M = H, Na oder K und n = 1 bis 3, entsprechend enthalten ist. Besonders bevorzugt ist mindestens eine OH-Gruppe des Rests G gemäß der obigen Formel durch eine Gruppe $-O-CH_2-COONa$ ersetzt.

Besonders bevorzugt wird ein Alkyloligoglykosidcarboxylat eingesetzt, in dem der Alkylrest ein Laurylrest ist. Speziell bevorzugt ist ein Laurylglucosidcarboxylat, wie es als Plantapon® LGC und Plantapon® LGC sorb von BASF SE erhältlich ist.

In den Alkylglykosiden der obigen Formel (A-2) leiten sich die Glykosid-Einheiten G vorzugsweise von Aldosen

bzw. Ketosen ab.

Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die als Ausgangsstoffe besonders bevorzugt eingesetzten Alkylglykoside sind daher die Alkylglucoside.

Die Indexzahl p gibt den Oligomerisierungsgrad, d.h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylglykosid eine analytische ermittelte rechnerische Größe, die meistens eine gebrochene Zahl darstellt. Vorzugsweise werden Alkylglykoside mit einem mittleren Oligomerisierungsgrad p von 1,1 bis 3,0 verwendet. Besonders bevorzugt sind solche Alkylglykoside, deren Oligomerisierungsgrad kleiner als 1,5 ist und insbesondere zwischen 1,1, und 1,4 liegt.

Der Alkylrest R leitet sich von primären Alkoholen mit 6 bis 22, vorzugsweise 12 bis 18 C-Atomen ab. Typische Beispiele sind Capronalkohol, Caprylalkohol, Caprinalkohol, Laurylalkohol, Myristylalkohol, Cetylalkohol, Stearylalkohol und Behenylakohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.

Neben den genannten Fettalkoholen können sich die Alkylglykoside auch von synthetischen primären Alkoholen mit 6 bis 22 C-Atomen, insbesondere den sogenannten Oxoalkoholen ableiten, die einen Anteil von 5 bis 40 Gew.-% verzweigter Isomeren aufweisen.

Besonders bevorzugte Alkylreste sind solche mit 8/10, 12/14, 8 bis 16, 12 bis 16 oder 16 bis 18 C-Atomen. Mischungen der Alkylreste ergeben sich bei einer Herstellung ausgehend von natürlichen Fetten und Ölen oder Mineralölen.

Verfahren zur Herstellung dieser Alkylglykoside sind beispielsweise in den amerikanischen Patentschriften US 3,547,828 und US 3,839,318 sowie der deutschen Patentanmeldung DE-A-37 23 826 beschrieben. Für Alkylpolyglykoside an sich kann beispielsweise auf die DE-A-100 27 975, DE-A-101 38 094 und DE-A-100 31 014 verwiesen werden.

Für eine weitere Diskussion der Alkyloligo/polyglykoside (APG) sei auf die internationale Patentveröffentlichung WO 03/013450 verwiesen.

Die Herstellung der erfindungsgemäß eingesetzten Alkyl- oder Alkenyl-Oligoglykosidcarboxylate, - phosphate, -sulfate oder-isethionate kann nach bekannten Verfahren erfolgen. Die Herstellung der Carboxylate erfolgt beispielsweise durch Umsetzung der Alkyloligoglykoside mit Salzen von Chlorcarbonsäuren in Gegenwart von Basen. Beispielsweise kann mit 2-Chloressigsäure-Natriumsalz in Gegenwart von NaOH umgesetzt werden. Bei der Umsetzung können sowohl die Hydroxylgruppen im Ring wie auch die $-CH_2-OH$-Gruppe umgesetzt werden. Der Umsetzungsgrad ist u.a. abhängig von der Stöchiometrie der Einsatzprodukte. Vorzugsweise werden die Alkyloligoglykoside zumindest an der $-CH_2-OH$-Gruppe umgesetzt, wobei optional ein Mittel eine oder mehreren der am Ring befindlichen Hydroxylgruppen umgesetzt werden können.

Weitere Hydroxylgruppen können beispielsweise auch verethert sein.

Die Herstellung der Isethionate ist beispielsweise in der WO 94/26857 beschrieben. Die Herstellung der Sulfate ist beispielsweise in der WO 93/10208 und WO 91/15192 beschrieben. In letzterer Schrift sind auch Gemische der APG-Sulfate mit u.a. Alkylsulfaten oder Alkylethersulfaten sowie weiteren Inhaltsstoffen beschrieben. Die Herstellung der Sulfate kann zudem wie in EP-A-0 186 242 beschrieben erfolgen. Beispielsweise kann das entsprechende Alkylglykosid mit gasförmigem Schwefeltrioxid oder mit Schwefelsäure, gefolgt von Neutralisierung, umgesetzt werden.

Die anionischen Tenside einschließlich der Fettsäureseifen können in Form ihrer Natrium-, Kalium- oder Magnesium- oder Ammoniumsalze vorliegen. Vorzugsweise liegen die anionischen Tenside in Form ihrer Natriumsalze und/oder Ammoniumsalze vor. Zur Neutralisation einsetzbare Amine sind vorzugsweise Cholin, Triethylamin, Monoethanolamin, Diethanolamin, Triethanolamin, Methylethylamin oder eine Mischung daraus, wobei Monoethanolamin bevorzugt ist. Wie bereits oben beschrieben, können die anionischen Tenside aber auch als Säure eingesetzt werden.

Als weiteren zwingenden Bestandteil enthält das erfindungsgemäße Flüssigwaschmittel mindestens ein Enzym mit einem pH-Optimum von < pH 7. Dabei ist es bevorzugt, wenn das erfindungsgemäße Flüssigwaschmittel mindestens ein Enzym mit einem pH-Optimum im pH-Bereich von pH 1 bis pH 6 enthält. Das pH-Optimum eines erfindungsgemäß eingesetzten Enzyms lässt sich im Allgemeinen durch Bestimmung der Enzymaktivität mittels dem Fachmann für entsprechendes Enzym / entsprechende Enzymklasse bekanntes Enzymessay in unterschiedlichen pH-Umgebungen ermitteln. Dabei ist im Rahmen der durchgeführten Versuchsreihe zur Ermittlung des pH-Optimums die pH-Umgebung der einzige Parameter, der variiert wird. Die Temperatur zur Durchführung der Enzymreaktion mit dem entsprechenden Substrat wird im Rahmen der Versuchsreihe zur Ermittlung des pH-Optimums bei 30°C gehalten.

Das erfindungsgemäße Flüssigwaschmittel enthält bevorzugt als Enzym mit einem pH-Optimum < pH 7 mindestens ein Enzym ausgewählt aus mindestens einem Enzym der Gruppe, die gebildet wird aus Protease mit einem pH-Optimum < pH 7, Amylase mit einem pH-Optimum < pH 7, Mannanase mit einem pH-Optimum < pH 7, Cellulase

mit einem pH-Optimum < pH 7. Dabei ist es erneut bevorzugt, wenn das besagte Enzym ein pH-Optimum im pH-Bereich von pH 1 bis pH 6 besitzt.

Es ist erfindungsgemäß besonders bevorzugt, wenn die erfindungsgemäßen Flüssigwaschmittel mindestens eine Protease mit einem pH-Optimum < pH 7, insbesondere im pH-Bereich von pH 1 bis pH 6, enthalten.

Eine Protease ist ein Enzym, das Peptidbindungen mittels Hydrolyse spaltet. Jedes der Enzyme aus der Klasse E.C. 3.4 fällt erfindungsgemäß darunter (umfassend jede der darunterfallenden dreizehn Unterklassen). Die EC-Nummer entspricht der Enzyme Nomenklatur 1992 der NC-IUBMB, Academic Press, San Diego, California, eingeschlossen der Ergänzungen 1 bis 5, publiziert in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650.

"Proteaseaktivität" liegt erfindungsgemäß vor, wenn das Enzym proteolytische Aktivität besitzt (EC 3.4). Verschiedenartige Proteaseaktivitäts-Typen sind bekannt: Die drei Haupttypen sind: Trypsin-artig, wobei eine Spaltung des Amidsubstrates nach den Aminosäuren Arg oder Lys bei P1 erfolgt; Chymotrypsin-artig, wobei eine Spaltung nach einer der hydrophoben Aminosäuren bei P1 erfolgt; und Elastase-artig, wobei eine Spaltung des Amidsubstrates nach Ala bei P1 erfolgt.

Die Proteaseaktivität kann nach der in Tenside, Band 7 (1970), S. 125-132 beschriebenen Methode ermittelt werden. Sie wird dementsprechend in PE (Protease-Einheiten) angegeben. Die Proteaseaktivität eines Enzyms lässt sich gemäß gängigen Standardmethoden, wie insbesondere unter Einsatz von BSA als Substrat (Rinderalbumin) und/oder mit der AAPF-Methode.

Es ist erfindungsgemäß bevorzugt, wenn die Protease mit einem pH-Optimum < pH 7, insbesondere im pH-Bereich von pH 1 bis pH 6, ausgewählt wird aus mindestens einer Endopeptidase, besonders bevorzugt aus Serin-Endopeptidasen (E.C. 3.4.21), Cystein-Endopeptidasen (E.C. 3.4.22), Aspartat-Endopeptidasen (E.C. 3.4.23) oder Mischungen daraus. Ganz besonders bevorzugt wird besagte Protease ausgewählt aus mindestens einer Cystein-Endopeptidase (E.C. 3.4.22) und/oder Aspartat-Endopeptidase (E.C. 3.4.23). Dabei ist es erfindungsgemäß erneut bevorzugt, wenn besagte Protease ausgewählt wird aus Protease aus *Aspergillus saitoi,* Protease aus *Aspergillus oryzae,* Bromelain, Pepsin, Rennin, Papain oder aus Mischungen daraus.

Optimierte Methoden zur Bestimmung des pH-Optimums dieser bevorzugten Proteasen sind der Literatur zu entnehmen und wurden zur Charakterisierung dieser Ausführungsformen eingesetzt, wobei die Temperatur während der Enzymreaktion auf 30°C eingestellt wurde:

Bromelain: Harrach et al., Journal of Protein Chemistry, Vol. 17, No.4, 1998, 351-361.
Pepsin: Brier et al., FEBS J., 274(23), 2007, 6152-6166.
Protease aus Aspergillus: Tsujita et al., Applied and environmental microbiology, Vol. 36, No.2, 1978, 237-242.
Rennin: Beldarrain et al., Biotechnol. Appl. Biochem., 31, 2000, 77-84.
Papain: Skelton, Acta Biocatalytica, Vol. 35, 1968, 270-274.)
Auf diese Druckschriften wird ausdrücklich und vollinhaltlich Bezug genommen.

Zusätzlich zu der erfindungsgemäß bevorzugt enthaltenen Protease enthält das erfindungsgemäße Flüssigwaschmittel besonders bevorzugt mindestens eine $\alpha$-Amylase mit einem pH-Optimum von < pH 7. Im Rahmen dieser Ausführungsform ist es bevorzugt, wenn das erfindungsgemäße Flüssigwaschmittel zusätzlich zu der besagten Protease mindestens eine $\alpha$-Amylase mit einem pH-Optimum im pH-Bereich von pH 1 bis pH 6 enthält. Das pH-Optimum der erfindungsgemäß bevorzugten $\alpha$-Amylase wird gemäß Chang et al., wie in Biochemistry and Molecular Biology International, Volume 36, No.1, May 1995, 185-193 beschrieben, bestimmt, wobei die Temperatur während der Enzymreaktion auf 30°C eingestellt wurde. Auf diese Druckschrift wird ausdrücklich und vollinhaltlich Bezug genommen.

Solche erfindungsgemäß bevorzugten $\alpha$-Amylasen werden von Mikroorganismen, das heißt Pilzen oder Bakterien, vor allem denen der Gattungen Aspergillus und Bacillus produziert und sekretiert. Ausgehend von diesen natürlichen Enzymen steht weiterhin eine nahezu unüberschaubare Fülle von Varianten zurVerfügung, die über Mutagenese abgeleitet worden sind und je nach Einsatzgebiet spezifische Vorteile aufweisen.

Bevorzugte erfindungsgemäße $\alpha$-Amylasen werden ausgewählt aus mindestens einer $\alpha$-Amylase aus *Aspergillus oryzae,* aus *Penicillium oxalicum,* aus Micrococcus sp. NS 211, aus *Bacillus sp. Ferdowsicous.*

Es ist erfindungsgemäß bevorzugt, wenn das erfindungsgemäße Flüssigwaschmittel als Enzym mit einem erfindungsgemäßen pH-Optimum mindestens eine Mannanase mit einem pH-Optimum von < pH 7 enthält. Im Rahmen dieser Ausführungsform ist es bevorzugt, wenn das erfindungsgemäße Flüssigwaschmittel mindestens eine Mannanase mit einem pH-Optimum im pH-Bereich von pH 1 bis pH 6 enthält.

Eine in der erfindungsgemäßen Zusammensetzung bevorzugt enthaltene Mannanase katalysiert im Rahmen ihrer Mannanase-Aktivität die Hydrolyse von 1,4-beta-D-mannosidischen Bindungen in Mannanen, Galactomannanen, Glucomannanen und Galactoglucomannanen. Bevorzugte erfindungsgemäße Mannanase-Enzyme werden gemäß Enzym Nomenklatur als E.C. 3.2.1.78 klassifiziert.

Die Mannanase-Aktivität eines Polypeptids bzw. Enzyms kann gemäß literaturbekannten Testmethoden bestimmt werden. Dabei wird beispielsweise eine Testlösung in Löcher mit 4 mm Durchmesser einer Agarplatte, enthaltend 0.2 Gew.-% AZGL Galactomannan (carob), i.e. Substrat für das endo-1,4-beta-D-Mannanase Essay, erhältlich unter Katalognummer I-AZGMA der Firma Megazyme (http://www.megazyme.com), eingebracht. Das pH-Optimum der erfindungsgemäß bevorzugten Mannanase wird ebenso gemäß o.g. endo-1,4-beta-D-Mannanase Essay bei unterschiedlichen pH-Werten bestimmt, wobei die Temperaturwährend der Enzymreaktion auf 30°C eingestellt wurde.

Eine bevorzugte erfindungsgemäße Mannanase ist eine Mannanase aus *Geobacillus tepidamans,* die beispielsweise von der Firma Danisco unter dem Handelsnamen Mannastar® erhältlich ist.

Es ist erfindungsgemäß bevorzugt, wenn das erfindungsgemäße Flüssigwaschmittel als Enzym mit einem erfindungsgemäßen pH-Optimum mindestens eine Cellulase mit einem pH-Optimum von < pH 7 enthält. Im Rahmen dieser Ausführungsform ist es bevorzugt, wenn das erfindungsgemäße Flüssigwaschmittel mindestens eine Mannanase mit einem pH-Optimum im pH-Bereich von pH 1 bis pH 6 enthält.

Die hierin beschriebenen Flüssigwaschmittel sind vorzugsweise homogene, niedrigviskose Lösungen der Zitronensäure in den übrigen Bestandteilen des Waschmittels, d.h. im Wesentlichen den eingesetzten Tensiden. "Homogen", wie in diesem Zusammenhang verwendet, bezieht sich auf molekulardisperse Lösungen und kolloidale Lösungen, die unter Standardbedingungen stabil sind, d.h. nach 24 h bei Raumtemperatur (25 °C) und 1013 mbar keine optisch wahrnehmbare Phasentrennung, einschließlich Präzipitation, aufweisen.

"Niedrigviskos", wie hierin verwendet, bedeutet, dass das wasserarme bis wasserfreie Flüssigwaschmittel wie hierin beschrieben eine Viskosität kleiner 15.000 mPas aufweist, vorzugsweise von 1.000 bis 5.000 mPas, noch bevorzugter von 2.000 bis 3.000 mPas (Brookfield Viskosimeter, Spindel Nr. 3 bei 20 °C).

Die Flüssigwaschmittel enthalten in bevorzugten Ausführungsformen zusätzlich ein oder mehrere nichtwässrige, organische Lösungsmittel. Dabei ist es bevorzugt, dass das Waschmittel mehr als 1 Gew.%, bevorzugt mehr als 5 Gew.% und insbesondere bevorzugt mehr als 10 Gew.%, jeweils bezogen auf die Gesamtmenge an Waschmittel, nichtwässrige Lösungsmittel enthält. Besonders bevorzugte flüssige Waschmittel enthalten - bezogen auf ihrGewicht- 1 bis 80 Gew.%, vorzugsweise 1 bis 65 Gew.%, bevorzugt 1 bis 50 Gew.%, besonders bevorzugt 5 bis 40 Gew.% und insbesondere 10 bis 30 Gew.% nichtwässrige Lösungsmittel.

Geeignete nichtwässrige Lösungsmittel umfassen ein- oder mehrwertige Alkohole, Alkanolamine oder Glykolether. Vorzugsweise werden die Lösungsmittel ausgewählt aus Ethanol, n-Propanol, i-Propanol, Butanolen, Glykol, Propandiol, Butandiol, Methylpropandiol, Glycerin, Glykolen, wie Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, 2,2-dimethyl-4-hydroxymetyl-1,3-dioxolan, Propylenglykol-t-butylether, Di-n-octylether sowie niedermolekularen Polyalkylenglykolen, wie PEG 400, sowie Mischungen dieser Lösungsmittel. In einer bevorzugten Ausführungsform wird das nichtwässrige Lösungsmittel ausgewählt aus der Gruppe bestehend aus Ethanol, n-Propanol, i-Propanol, Butanolen, Glykol, Propandiol, Butandiol, Methylpropandiol, Glycerin oder Gemischen davon. In einer bevorzugten Ausführungsform ist das nichtwässrige Lösungsmittel 1,2-Propandiol, Glycerin oder eine Mischung davon, ganz besonders bevorzugt Glycerin.

Generell kann der pH-Wert des Flüssigwaschmittels gemäß der Erfindung mittels üblicher pH-Regulatoren eingestellt werden. Allerdings ist der pH-Wert durch die Verwendung von Hydroxycarbonsäure mit 2 bis 8 Kohlenstoffatomen in den angegebenen Mengen üblicherweise in dem gewünschten Bereich.

Geeignete weitere pH-Stellmittel schließen Säuren und/oder Alkalien ein. Geeignete Säuren sind neben den besagten niedermolekularen Hydroxycarbonsäuren weitere organische Säuren wie die Essigsäure, Bernsteinsäure, Adipinsäure oder auch Amidosulfonsäure sowie Mischungen davon. Daneben können aber auch die Mineralsäuren Salzsäure, Schwefelsäure und Salpetersäure bzw. deren Mischungen eingesetzt werden. Geeignete Basen stammen aus der Gruppe der Alkali- und Erdalkalimetallhydroxide und -carbonate, insbesondere der Alkalimetallhydroxide, von denen Kaliumhydroxid und vor allem Natriumhydroxid bevorzugt ist. Auch flüchtiges Alkali kann verwendet werden, beispielsweise in Form von Ammoniak und/oder Alkanolaminen, die bis zu 9 C-Atome im Molekül enthalten können. Das Alkanolamin ist hierbei vorzugsweise ausgewählt aus der Gruppe bestehend aus Mono-, Di-, Triethanol- und -Propanolamin und deren Mischungen.

Zusätzlich kann das Waschmittel weitere Inhaltsstoffe enthalten, die die anwendungstechnischen und/oder ästhetischen Eigenschaften des Waschmittels weiter verbessern. Im Rahmen der vorliegenden Erfindung enthält das Waschmittel vorzugsweise zusätzlich einen oder mehrere Stoffe aus der Gruppe der Gerüststoffe/Komplexbildner, Bleichmittel, Elektrolyte, Parfüme, Parfümträger, Fluoreszenzmittel, Farbstoffe, Hydrotrope, Schauminhibitoren, Silikonöle, Antiredepositionsmittel, Vergrauungsinhibitore, Einlaufverhinderer, Knitterschutzmittel, Farbübertragungsinhibitoren, antimikrobielle Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, weichmachenden Komponenten sowie UV-Absorbern.

Enthält das erfindungsgemäße Waschmittel Bleichmittel, wird vorzugsweise Wasserstoffperoxid als Bleichmittel

verwendet.

Als Antiredepositionsmittel sind insbesondere Polymere auf Terephthalat-PEG Basis, wie sie beispielsweise unter dem Handelsnamen Texcare® kommerziell erhältlich sind, einsetzbar. Alternativ sind auch (Co)Polymere auf Basis von Polyethylenimin, Polyvinylacetat und Polyethylenglykol einsetzbar, bevorzugt in Mischungen mit Antiredepositionsmitteln.

Als zusätzliche Gerüststoffe sind insbesondere organische Gerüststoffe geeignet, beispielsweise die in Form ihrer Natriumsalze oder auch als Säuren einsetzbaren Polycarbonsäuren, wobei unter Polycarbonsäuren solche Carbonsäuren verstanden werden, die mehr als eine Säurefunktion tragen. Beispielsweise sind dies Adipinsäure, Glutarsäure, Maleinsäure, Fumarsäure, Aminocarbonsäuren, insbesondere Glutaminsäure-N,N-Diessigsäure (GLDA) und Methylglycin-N,N-Diessigsäure (MGDA), sowie Mischungen aus diesen. Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol. Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten. In flüssigen Waschmitteln werden bevorzugt lösliche Gerüststoffe, wie Acrylpolymere mit einer Molmasse von 1.000 bis 5.000 g/mol eingesetzt.

In verschiedenen Ausführungsformen der Erfindung kann das Flüssigwaschmittel ferner Oxalsäure in Mengen, bezogen auf das Gesamtgewicht des Flüssigwaschmittels, von bis zu 15 Gew.%, insbesondere 1-12 Gew.% enthalten. Die Oxalsäure kann die Wirksamkeit des Waschmittels bei der Entfernung von Rostflecken weiter steigern.

Die hierin beschriebenen Waschmittel können mittels im Stand der Technik bekannter Verfahren hergestellt werden. In einem Aspekt betrifft die Erfindung auch Verfahren zur Herstellung der hierin beschriebenen Waschmittel. Solche Verfahren können beispielsweise die Schritte umfassen:

1) Herstellen einer homogenen Lösung der Hydroxycarbonsäure mit 2 bis 8 Kohlenstoffatomen in dem mindestens einen nichtionischen Tensid oder einer Mischung aus dem mindestens einen nichtionischen Tensid und zusätzlich optional dem nichtwässrigen, organischen Lösungsmittel; und
2) Hinzufügen des erfindungsgemäß eingesetzten Enzyms und der optionalen übrigen Bestandteile des Waschmittels.

Hierbei kann die Herstellung in beliebiger Reihenfolge der Schritte 1) und 2) erfolgen.

Die hierin beschriebenen Waschmittel können in eine wasserlösliche Umhüllung gefüllt werden und somit Bestandteil einer wasserlöslichen Portion sein. Ist das Waschmittel in einer wasserlöslichen Umhüllung verpackt, ist es bevorzugt, dass der Gehalt an Wasser weniger als 10 Gew.-%, bezogen auf das gesamte Waschmittel, beträgt und dass anionischen Tenside, falls vorhanden, in Form ihrer Ammoniumsalze oder als freie Säuren vorliegen.

Die Neutralisation mit Aminen führt, anders als bei Basen wie NaOH oder KOH, nicht zu Bildung von Wasser. Somit können wasserarme Waschmittel hergestellt werden, die direkt für die Verwendung in wasserlöslichen Umhüllungen geeignet sind. Der Wasseranteil kann durch geeignete Salze, wie beispielsweise $MgSO_4$, $MgCO_3$, $Na_2SO_4$, $Na_2CO_3$ und Mischungen davon, reduziert werden.

Eine wasserlösliche Portion enthält neben dem Waschmittel eine wasserlösliche Umhüllung. Die wasserlösliche Umhüllung wird vorzugsweise durch ein wasserlösliches Folienmaterial gebildet.

Solche wasserlöslichen Portionen können entweder durch Verfahren des vertikalen Formfüllversiegelns (VFFS) oder Warmformverfahren hergestellt werden.

Das Warmformverfahren schließt im Allgemeinen das Formen einer ersten Lage aus einem wasserlöslichen Folienmaterial zum Bilden von Ausbuchtungen zum Aufnehmen einer Zusammensetzung darin, Einfüllen der Zusammensetzung in die Ausbuchtungen, Bedecken der mit der Zusammensetzung gefüllten Ausbuchtungen mit einer zweiten Lage aus einem wasserlöslichen Folienmaterial und Versiegeln der ersten und zweiten Lagen miteinander zumindest um die Ausbuchtungen herum ein.

Die wasserlösliche Umhüllung wird vorzugsweise aus einem wasserlöslichen Folienmaterial ausgewählt aus der Gruppe, bestehend aus Polymeren oder Polymergemischen gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein.

Die wasserlösliche Verpackung, umfassend das Waschmittel und die wasserlösliche Umhüllung, kann eine oder mehr Kammern aufweisen. Das flüssige Waschmittel kann in einer oder mehreren Kammern, falls vorhanden, der wasserlöslichen Umhüllung enthalten sein. Die Menge an flüssigem Waschmittel entspricht vorzugsweise der vollen oder halben Dosis, die für einen Waschgang benötigt wird.

Es ist bevorzugt, dass die wasserlösliche Umhüllung Polyvinylalkohol oder ein Polyvinylalkoholcopolymer enthält.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 g/mol, vorzugsweise von 20.000 bis 500.000 g/mol, besonders bevorzugt von 30.000 bis 100.000 g/mol und insbesondere von 40.000 bis 80.000 g/mol liegt.

Ein zur Herstellung der wasserlöslichen Umhüllung geeignetes Folienmaterial kann zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether Polymilchsäure, und/oder Mischungen der vorstehenden Polymere, zugesetzt sein.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist.

Ebenso bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Geeignete wasserlösliche Folien zum Einsatz in den Umhüllungen der wasserlöslichen Portionen sind Folien, die von der Firma MonoSol LLC beispielsweise unter der Bezeichnung M8630, C8400 oder M8900 vertrieben werden. Andere geeignete Folien umfassen Folien mit der Bezeichnung Solublon® PT, Solublon® GA, Solublon® KC oder Solublon® KL von der Aicello Chemical Europe GmbH oder die Folien VF-HP von Kuraray.

Die wasserlöslichen Verpackungen können eine im Wesentlichen formstabile kugelförmige und kissenförmige Ausgestaltung mit einer kreisförmigen, elliptischen, quadratischen oder rechteckigen Grundform aufweisen.

Die wasserlösliche Verpackung kann eine oder mehrere Kammern zur Bevorratung eines oder mehrerer Mittel aufweisen. Weist die wasserlösliche Portion zwei oder mehr Kammern auf, enthält mindestens eine Kammer ein flüssiges Waschmittel. Die weiteren Kammern können jeweils ein festes oder ein flüssiges Waschmittel enthalten.

Alle Sachverhalte, Gegenstände und Ausführungsformen, die für die Waschmittel beschrieben sind, sind auch auf die Verwendung sowie das Waschverfahren anwendbar und umgekehrt.

In einem weiteren Aspekt richtet sich die vorliegende Erfindung auf die Verwendung eines Flüssigwaschmittels wie hierin beschrieben zum Waschen von Textilien, insbesondere zur Entfernungen von Anschmutzungen, die auf Bestandteilen und Rückständen von Deodorantien basieren.

In einem weiteren Aspekt richtet sich die vorliegende Erfindung auf ein Waschverfahren, wobei ein Flüssigwaschmittel wie hierin beschrieben eingesetzt wird. Hierbei kann, wie bereits erwähnt, die Anschmutzung mit dem erfindungsgemäßen Flüssigwaschmittel vor dem eigentlichen Waschverfahren vorbehandelt werden und/oder zunächst eine Waschlösung bereitgestellt werden, die das erfindungsgemäße Flüssigwaschmittel enthält und die anschließend mit dem zu reinigendem Textil in Kontakt gebracht wird.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oderdass das Reinigungsgut in sonstiger Weise mit einem Waschmittel oder einer Lösung dieses Mittels behandelt wird.

In den beschriebenen Waschverfahren werden in verschiedenen Ausführungsformen der Erfindung Temperaturen von 60°C oder weniger, beispielsweise 40°C oder weniger, eingesetzt. Diese Temperaturangaben beziehen sich auf die in den Waschschritten eingesetzten Temperaturen.

Alle hierin im Zusammenhang mit den Waschmitteln der Erfindung beschriebenen Ausführungsformen, insbesondere im Hinblick auf die Spezifikation der Inhaltsstoffe, sind gleichermaßen auf die beschriebenen Verfahren und Verwendungen anwendbar und umgekehrt.

In bevorzugten Ausführungsformen der Erfindung enthalten die Flüssigwaschmittel bezogen auf ihr Gesamtgewicht 10 bis 25 Gew.-% Hydroxycarbonsäure ausgewählt aus Zitronensäure, Milchsäure oder deren Mischung, 40 bis 50 Gew.-% mindestens eines nichtionischen Tensids, vorzugsweise eines Fettalkoholethoxylats mit 5 bis 7 EO, noch bevorzugter eines C13 bis 15 Fettalkohols mit 7 EO, beispielsweise Lutensol® AO7, 1 bis 15 Gew.-% Wasser, 15 bis 25 Gew.-% Glycerin oder 1,2-Propandiol, vorzugsweise Glycerin und mindestens ein Enzym mit einem pH Optimum von pH < 7, insbesondere von pH 1 bis 6. Zusätzlich enthalten sein können 5 bis 8 Gew.-% Fettsäure, insbesondere C12 bis 18 Fettsäure und/oder 4 bis 5 Gew.-% Duftstoffe/Duftstoffzusammensetzung und/oder 6 bis 9 Gew.-% Antiredepositionsmittel, beispielsweise Texcare® SRN-170 (70%ig).

In bevorzugten Ausführungsformen der Erfindung enthalten die Flüssigwaschmittel bezogen auf ihr Gesamtgewicht 10 bis 25 Gew.-% Hydroxycarbonsäure ausgewählt aus Zitronensäure, Milchsäure oder deren Mischung, 40 bis 50 Gew.-% mindestens eines nichtionischen Tensids, vorzugsweise eines Fettalkoholethoxylats mit 5 bis 7 EO, noch bevorzugter eines C13 bis 15 Fettalkohols mit 7 EO, beispielsweise Lutensol® AO7, 1 bis 15 Gew.-% Wasser, 15 bis 25 Gew.-% Glycerin oder 1,2-Propandiol, vorzugsweise Glycerin und mindestens eine Protease (insbesondere die

bevorzugten (*vide supra*)) mit einem pH Optimum von pH < 7, insbesondere von pH 1 bis 6. Zusätzlich bevorzugt enthalten sein können 5 bis 8 Gew.-% Fettsäure, insbesondere C12 bis 18 Fettsäure und/oder 4 bis 5 Gew.-% Duftstoffe/Duftstoffzusammensetzung und/oder 6 bis 9 Gew.-% Antiredepositionsmittel, beispielsweise Texcare® SRN-170 (70%ig).

In bevorzugten Ausführungsformen der Erfindung enthalten die Flüssigwaschmittel bezogen auf ihr Gesamtgewicht 10 bis 25 Gew.-% Hydroxycarbonsäure ausgewählt aus Zitronensäure, Milchsäure oder deren Mischung, 40 bis 50 Gew.-% mindestens eines nichtionischen Tensids, vorzugsweise eines Fettalkoholethoxylats mit 5 bis 7 EO, noch bevorzugter eines C13 bis 15 Fettalkohols mit 7 EO, beispielsweise Lutensol® AO7, 1 bis 15 Gew.-% Wasser, 15 bis 25 Gew.-% Glycerin oder 1,2-Propandiol, vorzugsweise Glycerin und mindestens eine Cellulase mit einem pH Optimum von pH < 7, insbesondere von pH 1 bis 6. Zusätzlich bevorzugt enthalten sein können 5 bis 8 Gew.-% Fettsäure, insbesondere C12 bis 18 Fettsäure und/oder 4 bis 5 Gew.-% Duftstoffe/Duftstoffzusammensetzung und/oder 6 bis 9 Gew.-% Antiredepositionsmittel, beispielsweise Texcare® SRN-170 (70%ig).

In bevorzugten Ausführungsformen der Erfindung enthalten die Flüssigwaschmittel bezogen auf ihr Gesamtgewicht 10 bis 25 Gew.-% Hydroxycarbonsäure ausgewählt aus Zitronensäure, Milchsäure oder deren Mischung, 40 bis 50 Gew.-% mindestens eines nichtionischen Tensids, vorzugsweise eines Fettalkoholethoxylats mit 5 bis 7 EO, noch bevorzugter eines C13 bis 15 Fettalkohols mit 7 EO, beispielsweise Lutensol® AO7, 1 bis 15 Gew.-% Wasser, 15 bis 25 Gew.-% Glycerin oder 1,2-Propandiol, vorzugsweise Glycerin und mindestens eine Mannanase mit einem pH Optimum von pH < 7, insbesondere von pH 1 bis 6. Zusätzlich bevorzugt enthalten sein können 5 bis 8 Gew.-% Fettsäure, insbesondere C12 bis 18 Fettsäure und/oder 4 bis 5 Gew.-% Duftstoffe/Duftstoffzusammensetzung und/oder 6 bis 9 Gew.-% Antiredepositionsmittel, beispielsweise Texcare® SRN-170 (70%ig).

Beispiele

| | E1 | E2 | E3 | E4 | E5 |
|---|---|---|---|---|---|
| Niotensid mit 7 AO | 43,5 | 43,5 | 43,5 | 43,5 | 43,5 |
| C12-18 Kokosnussfettsäure | 6,2 | 6,2 | 6,2 | 6,2 | 6,2 |
| Glycerin | 18,7 | 18,7 | 18,7 | 18,7 | 18,7 |
| Zitronensäure*1 $H_2O$ | 18,7 | 18,7 | 18,7 | 18,7 | 18,7 |
| Parfüm | 4,0 | 4,0 | 4,0 | 4,0 | 4,0 |
| Ester aus Propandiol, Phthalsäure und alphamethyl-omega-hydroxypoly (oxy-1,2-ethandiyl) (CAS 139755-78-5) | 8,1 | 8,1 | 8,1 | 8,1 | 8,1 |
| Protease 1 | 0,8 | - | - | - | - |
| Protease 2 | - | 0,8 | - | - | - |
| Protease 3 | - | - | 0,8 | - | - |
| Protease 4 | - | - | - | 0,8 | - |
| Protease 5 | - | - | - | - | 0,8 |
| pH-Wert einer 1 Gew.-%-igen Lösung in destilliertem Wasser bei 25°C | 4,6 | 4,6 | 4,6 | 4,6 | 4,6 |
| Viskositätsmessung [mPas] | 2900 | 2900 | 2900 | 2900 | 2900 |
| Optisches Erscheinungsbild | klar | klar | klar | klar | klar |
| Protease 1: Papain aus *Carica Papaya* (Sigma)<br>Protease 2: Bromelain aus dem Stamm der Ananaspflanze (Sigma)<br>Protease 3: Pepsin aus *porcine gastric mucosa* (Sigma)<br>Protease 4: Rennin aus *Mucor miehei* (Sigma)<br>Protease 5: Protease aus *Aspergillus Saitoi* Typ XIII (Sigma) | | | | | |

Die obigen Angaben der Bestandteile sind in Gew.% angegeben. Die Formulierungen wurden in 1 L Wasser gelöst und auf ihr Aussehen untersucht. Die Viskositätsmessungen wurden mit einem Brookfield Viskositätsmesser, Spindel Nr. 3 bei 20 °C durchgeführt.

## Patentansprüche

1. Flüssigwaschmittel, enthaltend bezogen auf das Gesamtgewicht des Flüssigwaschmittels

   (i) 10 bis 30 Gew.%, insbesondere 15 bis 25 Gew.% mindestens einer Hydroxycarbonsäure mit 2 bis 8 Kohlenstoffatomen und
   (ii) 20 bis 55 Gew.%, insbesondere 35 bis 50 Gew.% mindestens eines nichtionischen Tensides enthält,
   (iii) 0 bis 20 Gew.-% Wasser,
   (iv) mindestens ein Enzym mit einem pH-Optimum bei 30°C von pH < 7 wobei das Flüssigwaschmittel einen pH-Wert (1%ige (Gew.-%) Lösung des Waschmittels in destilliertem Wasser bei 25°C im Bereich 2 bis 5 aufweist.

2. Flüssigwaschmittel nach Anspruch 1, **dadurch gekennzeichnet, dass** als besagte Hydroxycarbonsäure mindestens eine, aliphatische Hydroxycarbonsäure mit 3 bis 6 Kohlenstoffatomen enthalten ist, wobei besagte Hydroxycarbonsäure linear oder verzweigt ist.

3. Flüssigwaschmittel nach Anspruch 1, **dadurch gekennzeichnet, dass** als besagte Hydroxycarbonsäure mindestens eine Hydroxycarbonsäure ausgewählt aus mindestens einem Vertreter der Gruppe enthalten ist, die gebildet wird aus Zitronensäure, Milchsäure, Weinsäure, Hydroxybernsteinsäure, Salicylsäure.

4. Flüssigwaschmittel nach Anspruch 1 bis 3, **dadurch gekennzeichnet, dass** das mindestens eine nichtionische Tensid ein Fettalkoholethoxylat umfasst.

5. Flüssigwaschmittel nach einem der Ansprüche 1 bis 4, **dadurch gekennzeichnet, dass** das besagte Enzym ausgewählt wird aus mindestens einem Enzym der Gruppe, die gebildet wird aus Protease mit einem pH-Optimum bei 30°C < pH 7, Amylase mit einem pH-Optimum bei 30°C < pH 7, Mannanase mit einem pH-Optimum bei 30°C < pH 7, Cellulase mit einem pH-Optimum bei 30°C < pH 7.

6. Flüssigwaschmittel nach einem der Ansprüche 1 bis 5, **dadurch gekennzeichnet, dass** es mindestens ein Enzym mit einem pH-Optimum bei 30°C von pH 1 bis pH 6 enthält.

7. Flüssigwaschmittel nach einem der Ansprüche 1 bis 6, **dadurch gekennzeichnet, dass** das besagte Enzym mindestens eine Protease ist, ausgewählt aus mindestens einer Endopeptidase, bevorzugt aus Serin-Endopeptidasen (E.C. 3.4.21), Cystein-Endopeptidasen (E.C. 3.4.22), Aspartat-Endopeptidasen (E.C. 3.4.23) oder Mischungen daraus.

8. Flüssigwaschmittel nach einem der Ansprüche 1 bis 7, **dadurch gekennzeichnet, dass** das besagte Enzym mindestens eine Protease ist, ausgewählt aus Protease aus *Aspergillus saitoi*, Protease aus *Aspergillus oryzae,* Bromelain, Pepsin, Rennin, Papain oder aus Mischungen daraus.

9. Flüssigwaschmittel nach einem der Ansprüche 1 bis 8, **dadurch gekennzeichnet, dass** das Flüssigwaschmittel bezogen auf das Gesamtgewicht ferner 1 bis 50 Gew.%, vorzugsweise 10 bis 30 Gew.%, nichtwässriges, organisches Lösungsmittel enthält, insbesondere ausgewählt aus der Gruppe bestehend aus 1,2-Propandiol, Glycerin, 2,2-dimethyl-4-hydroxymethyl-1,3-dioxolan und Gemischen davon.

10. Flüssigwaschmittel nach einem der Ansprüche 1 bis 9, **dadurch gekennzeichnet, dass** das Flüssigwaschmittel homogen ist und/oder eine Viskosität (Brookfield-Viskosimeter, Spindel Nr. 3, 20°C) kleiner 15.000 mPas, insbesondere im Bereich 1.000 bis 5.000 mPas, besonders bevorzugt im Bereich von 2.000 bis 3.000 mPas aufweist.

11. Flüssigwaschmittel nach einem der Ansprüche 1 bis 10, **dadurch gekennzeichnet, dass** das Mittel ferner mindestens einen weiteren Bestandteil ausgewählt aus der Gruppe bestehend aus weiteren Tensiden, weiteren Enzymen, Gerüststoffen, Bleichmitteln, Elektrolyten, Parfümen, Parfümträgern, Fluoreszenzmitteln, Farbstoffen, Hydrotropen, Schauminhibitoren, Silikonölen, Antiredepositionsmitteln, Vergrauungsinhibitoren, Einlaufverhinderern, Knitterschutzmitteln, Farbübertragungsinhibitoren, antimikrobiellen Wirkstoffen, Germiziden, Fungiziden, Antioxidantien, Konservierungsmitteln, Korrosionsinhibitoren, Antistatika, Bittermitteln, Bügelhilfsmitteln, Phobier- und Imprägniermitteln, Quell- und Schiebefestmitteln, weichmachenden Komponenten sowie UV-Absorbern enthält.

12. Flüssigwaschmittel nach einem der Ansprüche 1 bis 11, **dadurch gekennzeichnet, dass** das Mittel in einer wasserunlöslichen, wasserlöslichen oder wasserdispergierbaren Verpackung befindet, insbesondere in einer Polyvinylalkohol-haltigen Folie.

**13.** Verwendung eines Flüssigwaschmittels nach einem der Ansprüche 1 bis 12 zum Waschen von Textilien, insbesondere zur Entfernungen von Anschmutzungen, die auf Bestandteilen und Rückständen von Deodorantien, Rost, Beeren, Tee und Rotwein basieren.

**14.** Waschverfahren zum Waschen von Textilien, **dadurch gekennzeichnet, dass** ein Flüssigwaschmittel nach einem der Ansprüche 1 bis 12 eingesetzt wird.

**Claims**

**1.** A liquid washing agent containing, based on the total weight of the liquid washing agent,

(i) 10 to 30 wt.%, in particular 15 to 25 wt.%, of at least one hydroxycarboxylic acid having 2 to 8 carbon atoms and
(ii) 20 to 55 wt.%, in particular 35 to 50 wt.%, of at least one non-ionic surfactant,
(iii) 0 to 20 wt.% water,
(iv) at least one enzyme having an optimum pH at 30 °C of pH < 7,

wherein the liquid washing agent has a pH (1% (wt.%) solution of the washing agent in distilled water at 25 °C) in the range of 2 to 5.

**2.** The liquid washing agent according to claim 1, **characterized in that** at least one aliphatic hydroxycarboxylic acid having 3 to 6 carbon atoms is contained as said hydroxycarboxylic acid, said hydroxycarboxylic acid being linear or branched.

**3.** The liquid washing agent according to claim 1, **characterized in that** at least one hydroxycarboxylic acid selected from at least one representative of the group formed by citric acid, lactic acid, tartaric acid, hydroxysuccinic acid and salicylic acid is contained as said hydroxycarboxylic acid.

**4.** The liquid washing agent according to claim 1 to 3, **characterized in that** the at least one non-ionic surfactant comprises a fatty alcohol ethoxylate.

**5.** The liquid washing agent according to one of claims 1 to 4, **characterized in that** said enzyme is selected from at least one enzyme of the group formed by protease having an optimum pH at 30 °C < pH 7, amylase having an optimum pH at 30 °C < pH 7, mannanase having an optimum pH at 30 °C < pH 7, and cellulase having an optimum pH at 30 °C < pH 7.

**6.** The liquid washing agent according to one of claims 1 to 5, **characterized in that** it contains at least one enzyme having an optimum pH at 30 °C of pH 1 to pH 6.

**7.** The liquid washing agent according to one of claims 1 to 6, **characterized in that** said enzyme is at least one protease selected from at least one endopeptidase, preferably from serine endopeptidases (EC 3.4.21), cysteine endopeptidases (EC 3.4.22), aspartate endopeptidases (EC 3.4.23) or mixtures thereof.

**8.** The liquid washing agent according to one of claims 1 to 7, **characterized in that** said enzyme is at least one protease selected from protease from *Aspergillus saitoi,* protease from *Aspergillus oryzae,* bromelain, pepsin, rennin, papain or mixtures thereof.

**9.** The liquid washing agent according to one of claims 1 to 8, **characterized in that** the liquid washing agent further contains, based on the total weight, 1 to 50 wt.%, preferably 10 to 30 wt.%, non-aqueous organic solvent, in particular selected from the group consisting of 1,2-propanediol, glycerol, 2,2-dimethyl-4-hydroxymethyl-1,3-dioxolane and mixtures thereof.

**10.** The liquid washing agent according to one of claims 1 to 9, **characterized in that** the liquid washing agent is homogeneous and/or has a viscosity (Brookfield viscometer, spindle no. 3, 20 °C) of less than 15,000 mPas, in particular in the range of 1,000 to 5,000 mPas, particularly preferably in the range of 2,000 to 3,000 mPas.

**11.** The liquid washing agent according to one of claims 1 to 10, **characterized in that** the agent further contains at least one further component selected from the group consisting of further surfactants, further enzymes, builders, bleaching agents, electrolytes, perfumes, perfume carriers, fluorescing agents, dyes, hydrotropic substances, suds suppressors, silicone oils, anti-redeposition agents, graying inhibitors, anti-shrink agents, anti-crease agents, dye transfer inhibitors, antimicrobial active ingredients, germicides, fungicides, antioxidants, preservatives, corrosion inhibitors, antistatic agents, bittering agents, ironing aids, repellents and impregnating agents, anti-swelling and anti-slip agents, softening components and UV absorbers.

**12.** The liquid washing agent according to one of claims 1 to 11, **characterized in that** the agent is in a water-insoluble, water-soluble or water-dispersible packaging, in particular in a film which contains polyvinyl alcohol.

**13.** The use of a liquid washing agent according to one of claims 1 to 12 for washing textiles, in particular for removing stains which are based on components and residues of deodorants, rust, berries, tea and red wine.

**14.** A washing method for washing textiles, **characterized in that** a liquid washing agent according to one of claims 1 to 12 is used.

**Revendications**

**1.** Détergent liquide, contenant, par rapport au poids total du détergent liquide

(i) 10 à 30 % en poids, en particulier 15 à 25 % en poids, d'au moins un acide hydroxycarboxylique comportant 2 à 8 atomes de carbone et
(ii) 20 à 55 % en poids, en particulier 35 à 50 % en poids, d'au moins un tensioactif non ionique,
(iii) 0 à 20 % en poids d'eau,
(iv) au moins une enzyme comportant un pH optimal pH < 7 à 30 °C,

le détergent liquide présentant un taux de pH (1 % (% en poids) de solution du détergent dans la plage de 2 à 5 dans de l'eau distillée à 25 °C.

**2.** Détergent liquide selon la revendication 1, **caractérisé en ce qu'**il contient, en tant que ledit acide hydroxycarboxylique, au moins un acide hydroxycarboxylique aliphatique comportant 3 à 6 atomes de carbone, ledit acide hydroxycarboxylique étant linéaire ou ramifié.

**3.** Détergent liquide selon la revendication 1, **caractérisé en ce qu'**il contient, en tant que ledit acide hydroxycarboxylique, au moins un acide hydroxycarboxylique choisi parmi au moins un représentant du groupe constitué par l'acide citrique, l'acide lactique, l'acide tartrique, l'acide hydroxysuccinique et l'acide salicylique.

**4.** Détergent liquide selon l'une des revendications 1 à 3, **caractérisé en ce que** l'au moins un tensioactif non ionique comprend un éthoxylate d'alcool gras.

**5.** Détergent liquide selon l'une des revendications 1 à 4, **caractérisé en ce que** ladite enzyme est choisie parmi au moins une enzyme du groupe constitué par la protéase comportant un pH optimal < pH 7 à 30 °C, l'amylase comportant un pH optimal < pH 7 à 30 °C, la mannanase comportant un pH optimal < pH 7 à 30 °C et la cellulase comportant un pH optimal < pH 7 à 30 °C.

**6.** Détergent liquide selon l'une des revendications 1 à 5, **caractérisé en ce qu'**il contient au moins une enzyme comportant un pH optimal de pH 1 à pH 6 à 30 °C.

**7.** Détergent liquide selon l'une des revendications 1 à 6, **caractérisé en ce que** ladite enzyme est au moins une protéase choisie parmi au moins une endopeptidase, de préférence parmi les endopeptidases à sérine (EC 3.4.21), les endopeptidases à cystéine (EC 3.4.22), les endopeptidases à aspartate (EC 3.4.23) ou leurs mélanges.

**8.** Détergent liquide selon l'une des revendications 1 à 7, **caractérisé en ce que** ladite enzyme est au moins une protéase choisie parmi la protéase d'*Aspergillus saitoi*, la protéase d'*Aspergillus oryzae*, la broméline, la pepsine, la rennine, la papaïne ou leurs mélanges.

**9.** Détergent liquide selon l'une des revendications 1 à 8, **caractérisé en ce que** le détergent liquide contient en outre, par rapport au poids total, 1 à 50 % en poids, de préférence 10 à 30 % en poids, d'un solvant organique non aqueux, en particulier choisi dans le groupe constitué par le 1,2-propanediol, la glycérine, le 2,2-diméthyl-4-hydroxyméthyl-1,3-dioxolane et leurs mélanges.

**10.** Détergent liquide selon l'une des revendications 1 à 9, **caractérisé en ce que** le détergent liquide est homogène et/ou présente une viscosité (viscosimètre Brookfield, broche n°3, 20 °C) inférieure à 15 000 MPa, en particulier dans la plage de 1 000 à 5 000 MPa, de manière particulièrement préférée dans la plage de 2 000 à 3 000 MPa.

**11.** Détergent liquide selon l'une des revendications 1 à 10, **caractérisé en ce que** le détergent contient en outre au moins un autre constituant choisi dans le groupe constitué par d'autres tensioactifs, d'autres enzymes, adjuvants, agents de blanchiment, électrolytes, parfums, supports de parfum, agents fluorescents, colorants, hydrotropes, inhibiteurs de mousse, huiles de silicone, agents anti-redéposition, inhibiteurs de grisaillement, agents anti-rétrécissement, agents anti-froissage, inhibiteurs de transfert de couleur, agents actifs antimicrobiens, germicides, fongicides, antioxydants, conservateurs, inhibiteurs de corrosion, antistatiques, agents amers, auxiliaires de repassage, agents de répulsion et d'imprégnation, agents gonflants et antidérapants, composants adoucissants ainsi qu'absorbeurs d'UV.

**12.** Détergent liquide selon l'une des revendications 1 à 11, **caractérisé en ce que** le détergent se trouve dans un emballage insoluble dans l'eau, soluble dans l'eau ou dispersible dans l'eau, en particulier dans un film contenant un alcool polyvinylique.

**13.** Utilisation d'un détergent liquide selon l'une des revendications 1 à 12 pour le lavage de textiles, en particulier pour l'élimination de salissures à base de constituants et de résidus de déodorants, de rouille, de baies, de thé et de vin rouge.

**14.** Procédé de lavage pour le lavage de textiles, **caractérisé en ce qu'**un détergent liquide selon l'une des revendications 1 à 12 est utilisé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012149325 A
- US 3547828 A
- US 3839318 A
- DE 3723826 A
- DE 10027975 A
- DE 10138094 A
- DE 10031014 A
- WO 03013450 A
- WO 9426857 A
- WO 9310208 A
- WO 9115192 A
- EP 0186242 A


### In der Beschreibung aufgeführte Nicht-Patentliteratur


- **CHANG et al.** *Biochem. Mol. Biol. Int.,* Mai 1995, 185-193
- **EUGEN SCHOLZ.** *Angewandte Chemie,* 1935, vol. 48, ISBN 3-540-12846-8, 394-396
- **GRIFFIN.** Classification of surface active agents by HLB. *J. Soc. Cosmet. Chem.,* 1949, vol. 1
- *Eur. J. Biochem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- *Tenside,* 1970, vol. 7, 125-132
- **HARRACH et al.** *Journal of Protein Chemistry,* 1998, vol. 17 (4), 351-361
- **BRIER et al.** *FEBS J.,* 2007, vol. 274 (23), 6152-6166
- **TSUJITA et al.** *Applied and environmental microbiology,* 1978, vol. 36 (2), 237-242
- **BELDARRAIN et al.** *Biotechnol. Appl. Biochem.,* 2000, vol. 31, 77-84
- **SKELTON.** *Acta Biocatalytica,* 1968, vol. 35, 270-274
- **CHANG et al.** *Biochemistry and Molecular Biology International,* Mai 1995, vol. 36 (1), 185-193
- *CHEMICAL ABSTRACTS,* 139755-78-5